Europäisches Patentamt

(19) European Patent Office

Office européen des brevets

(11) Numéro de publication : **0 220 097 B1**

# (12) FASCICULE DE BREVET EUROPEEN

(45) Date de publication du fascicule du brevet :
02.01.91 Bulletin 91/01

(21) Numéro de dépôt : **86402074.8**

(22) Date de dépôt : **22.09.86**

(51) Int. Cl.⁵ : **A61F 2/24**

(54) **Valve cardiaque prothétique.**

(30) Priorité : **23.09.85 FR 8514051**

(43) Date de publication de la demande :
**29.04.87 Bulletin 87/18**

(45) Mention de la délivrance du brevet :
**02.01.91 Bulletin 91/01**

(84) Etats contractants désignés :
**AT BE CH DE FR GB IT LI LU NL SE**

(56) Documents cités :
**WO-A-82/03981**
**FR-A- 1 503 297**
**GB-A- 2 051 308**
**US-A- 4 078 268**
**US-A- 4 306 319**
**US-A- 4 417 360**
**US-A- 4 488 318**

(73) Titulaire : **AVIONS MARCEL**
**DASSAULT-BREGUET AVIATION**
**33, Rue du Professeur Victor Pauchet**
**F-92420 Vaucresson (FR)**
Titulaire : **Lapeyre, Didier, Dr.**
**Chaignes**
**F-27120 Pacy sur Eure (FR)**

(72) Inventeur : **Perrier, Philippe**
**4, rue A. Daudet**
**F-78860 St-Nom-La-Breteche (FR)**
Inventeur : **Lapeyre, Didier**
**Chaignes**
**F-27120 Pacy S/Eure (FR)**

(74) Mandataire : **Orès, Bernard et al**
**Cabinet ORES 6, Avenue de Messine**
**F-75008 Paris (FR)**

# Description

La présente invention est relative à une valve cardiaque prothétique destinée à être implantée chez l'homme pour le remplacement d'une valve cardiaque naturelle endommagée ou ayant un mauvais fonctionnement.

Plusieurs prothèses de valves cardiaques ont été proposées dans l'Art antérieur. Dans l'ouvrage de E.A. LEFRAK et A. STARR, édité en 1979 par Appleton-Century-Crofts, New York, et ayant pour titre : "CARDIAC VALVE PROSTHESES", sont présentés les aspects historiques, hémodynamiques et cliniques de différentes valves cardiaques prothétiques mécaniques et biologiques. Parmi les valves cardiaques mécaniques (auxquelles se réfère notre analyse de l'Art antérieur parce que les valves prothétiques biologiques sortent du cadre de l'invention), on se limite à rappeler ici certaines des solutions comportant au moins deux clapets ou volets laissant s'écouler le sang dans un sens et l'empêchant de refluer dans le sens inverse.

Le brevet américain S. MILO, n° 3 938 197, décrit une valve cardiaque artificielle à écoulement central, à savoir présentant la zone centrale complètement dégagée en position d'ouverture. Cette valve comporte une pluralité de volets, notamment cinq, qui ont une forme sensiblement triangulaire et dont la face inférieure est concave sur environ les 4/5 inférieurs de sa hauteur, tandis qu'elle est convexe sur environ le 1/5 supérieur de ladite hauteur, lesquels volets sont articulés chacun autour d'un segment périphérique constitué par l'assemblage mâle/femelle de deux éléments, lequel assemblage est stabilisé à l'aide d'une vis qui est enfilée dans un trou périphérique ménagé dans un élément parallèlement à l'axe de pivotement du volet correspondant et qui se visse dans l'autre élément.

Le brevet américain Z.C. POSSIS, n° 4 078 268, décrit une valve cardiaque prothétique comportant, dans un premier mode de réalisation, deux clapets pivotant chacun autour d'un axe solidaire du clapet correspondant et constitué par deux projections faisant axe, disposées sur les deux côtés latéraux du clapet à une distance de son bord d'attaque égale à 1/3 de la hauteur du clapet, le mouvement de rotation de chaque clapet étant limité par des projections appropriées faisant saillie de la jante (ou base ou corps) de support des volets, vers le passage central de la valve.

Selon un deuxième mode de réalisation, le brevet POSSIS susdit décrit des clapets dont le mouvement de rotation entre les positions de fermeture et d'ouverture et vice versa, est assuré, sous l'action de la pulsation cardiaque, par des moyens de pivotement et de butée comprenant chacun deux paires de projections qui font légèrement saillie de la base de la valve vers le passage central et qui définissent ensemble un axe de rotation virtuel. Chaque paire de projections est constituée par une projection supérieure pourvue d'une portion de pivotement et d'une face de butée sensiblement verticale, et par une projection inférieure légèrement décalée par rapport à la portion supérieure et elle aussi pourvue d'une portion de pivotement et d'une face de butée inclinée vers l'intérieur et vers le haut. Chaque clapet est compris entre deux paires de projections, dont la projection supérieure coopère avec une oreille latérale faisant saillie de la face supérieure du clapet correspondant et disposée en dessous par rapport à ladite projection supérieure.

En ce qui concerne la base de support des volets décrits dans le brevet POSSIS, celle-ci est réalisée par vissage ou par emboîtement/encliquetage d'une portion interne dans une portion externe, la portion interne pouvant être constituée par deux segments semi-circulaires dans le cas où on effectue l'assemblage par emboîtement/encliquetage.

De plus, la distance de chacun des deux axes de rotation réels ou virtuels, par rapport à l'axe de la valve est comprise entre environ 75% et 65% du rayon du passage central délimité par la base de support, en sorte que ce dernier est amplement dégagé, sans toutefois être dégagé de façon optimale.

Le brevet français CARPENTIER n° 2 331 997, correspondant au brevet américain n° 4 159 543, décrit une valve cardiaque prothétique du type comprenant une base de support sensiblement circulaire, pourvue d'au moins un siège, et deux clapets mobiles sensiblement demi-circulaires pouvant reposer sur le siège.

Chaque clapet de la valve CARPENTIER coopère avec des moyens permettant la rotation et la translation des deux clapets, et notamment la rotation d'un angle inférieur à 90° et la translation parallèlement à l'axe de la base de support. Les moyens de rototranslation sont constitués, pour chaque clapet, par un axe transversal disposé à proximité d'un diamètre de la base et par des picots, notamment en forme d'arêtes profilées, disposés alternativement de part et d'autre par rapport à l'axe correspondant et faisant saillie de la face externe du clapet. La translation est assurée par le fait que, lorsqu'on considère la valve en position de fermeture et donc avec chaque clapet reposant sur le siège correspondant, entre l'axe de rotation et la face externe du clapet existe une certaine distance, ce qui empêche le contact permanent entre chaque clapet et l'arbre correspondant.

On connaît également la demande de brevet français n° 2 543 429, au nom de l'UNIVERSITE PIERRE ET MARIE CURIE et ayant comme inventeur encore une fois M. CARPENTIER, dans laquelle on apporte des perfectionnements notamment à la valve cardiaque prothétique décrite dans le brevet français CARPENTIER susdit n° 2 331 997 : il s'agit de pourvoir chaque clapet et/ou le siège correspondant de la valve d'un dispositif connu en soi, capable d'assurer une ouverture contrôlée de la valve lorsque la pression sanguine est équilibrée sur les deux faces, interne et externe, des clapets mobiles ; cela signifie que, dans ces conditions d'équilibre des pressions, la valve est légèrement ouverte. Le dispositif, connu en soi, proposé par CARPENTIER pour contrôler l'ouverture de la valve est constitué pour chaque clapet par deux aimants ou électro-aimants dont l'un est disposé sur le clapet tandis que l'autre est disposé sur le siège correspondant. En alternative, le dispositif peut être constitué par un ressort fixé au siège.

En outre, le brevet américain n° 847 780 D.W. HANSON et al., correspondant au brevet français n° 2 407 709, décrit une prothèse de valve cardiaque du type comprenant un corps pourvu d'un passage central d'écoulement du sang, des moyens de réglage de cet écoulement, qui pivotent sous l'action de l'écoulement sanguin entre une position de fermeture et une position d'ouverture, et des moyens d'articulation de ces moyens de réglage. La valve HANSON et al. comporte des moyens d'articulation comprenant des cavités ménagées dans ledit corps et présentant une surface de portée en forme de surface de révolution et des moyens de réglage pourvus de saillies destinées à pénétrer dans les cavités et à balayer ladite surface de butée avec l'extrémité qui est en contact avec cette surface.

On connaît aussi le brevet américain L.C. MEYER, n° 3 589 392, qui décrit une valve cardiaque artificielle dont les volets présentent la face interne concave (sensiblement en forme de cuillère) et sont articulés (pivotent) autour d'une portion flexible arquée qui est supportée par la base de la valve ; toutefois, la valve MEYER présente un encombrement en hauteur qui est exagérément important, ce qui est dû notamment à la conception des moyens de pivotement utilisés pour les clapets.

Mise à part la valve MEYER pour les raisons susdites, et en particulier parce qu'elle n'est pas une valve du type dit "low-profile", il est clair que chacune des valves cardiaques prothétiques de l'Art antérieur présente des avantages et des inconvénients qui lui sont propres, notamment :
· en ce qui concerne la valve MILO, si d'un côté celle-ci assure un écoulement central du sang, par contre elle présente des zones de stagnation du sang sous lesdits segments périphériques d'articulation des volets, à savoir que les éléments cellulaires du sang ont tendance à rester confinés sous ces segments, ce qui produit un début de coagulation et donc à la longue des effets de thrombose ;
· en ce qui concerne la valve POSSIS, si celle-ci élimine les problèmes de stagnation du sang posés par la valve MILO, toutefois le passage central d'écoulement du sang n'est pas encore dégagé au maximum des possibilités et les volets sont inclinés vers l'axe, en position d'ouverture, en sorte que les problèmes de turbulence -même s'ils sont réduits-sont encore importants ; en outre, la solution constructive de la base de support des clapets réalisée en plusieurs éléments présente les risques potentiels de séparation de ces éléments et de formation de zones de stagnation du sang ;
· en ce qui concerne la valve CARPENTIER décrite dans le brevet français n° 2 331 997, si celle-ci évite l'établissement de contacts permanents entre les parties fixes et les parties mobiles de la prothèse et donc réduit la formation de caillots, par contre la position sensiblement central des clapets et leur inclinaison relativement importante par rapport à la verticale sont source de turbulences dans la partie centrale de l'écoulement, lesquelles turbulences sont d'autant plus importantes et gênantes que l'écoulement est plus rapide à cet endroit là, en sorte que l'avantage précédent est pratiquement annulé et les risques de coagulation existent également dans ce cas ;
· en ce qui concerne la valve CARPENTIER décrite dans le brevet français n° 2 543 429, il y a lieu de souligner que celle-ci privilégie les performances à l'ouverture alors que, dans la pratique, c'est plutôt le comportement de la valve à la fermeture qui pose des problèmes, notamment dus à la régurgitation sanguine lors du retour de l'écoulement sanguin en sens inverse ;
· en ce qui concerne la valve HANSON et al., les effets nuisibles (écrasement et donc destruction) sur les globules rouges du sang restent encore importants du fait de l'action de friction entre lesdites saillies des clapets et lesdites cavités de logement de ces saillies.

En outre, d'une façon générale, la conception de toutes les valves prothétiques de l'Art antérieur est telle que les articulations sont soumises à des efforts très importants qui sont responsables de l'usure prématurée des valves existantes. La présente invention a donc également pour but de pourvoir à une valve prothétique dont les articulations sont faiblement sollicitées, soignant ainsi un aspect vital dans la conception des prothèses valvulaires cardiaques, comme le souligne justement le chirurgien D. LIOTTA de l'HOSPITAL ITALIANO de Buenos Aires, en Argentine, à la page 49 du TEXAS HEART INSTITUTE JOURNAL, vol. 12, n° 1, de mars 1985.

La présente invention a pour but de pourvoir à une valve cardiaque prothétique qui répond mieux aux nécessités de la pratique que les prothèses de valves cardiaques antérieurement connues, notamment en ce que :
· en position d'ouverture :
– la perturbation (turbulence) de l'écoulement du sang dans le sens direct est la moindre possible ;
– les pertes de charge de pression sont minimales ;
– les pertes de charge par frottement sont également minimisées ;
· les clapets, du fait de leur disposition sensiblement parallèle à l'écoulement, ne subissent pas ou subissent en moindre quantité les efforts de pression, ce qui évite la création de tourbillons au voisinage des clapets ;

· les contraintes mécaniques locales et l'usure sont limitées dans une grande mesure parce que les efforts plus importants sont répartis sur les portions massives de la valve (base de support et clapets) dont l'usure est généralement très faible ;

· le bruit de fonctionnement est sensiblement réduit ;

· la fermeture des moyens d'obturation peut être anticipée sur le retour de l'écoulement sanguin, ce qui permet de minimiser les problèmes de régurgitation ;

· les contraintes agissant sur les moyens de retenue (butée) des clapets ne sont pas amplifiées par effet de levier ;

· l'ouverture des moyens d'obturation se fait naturellement dès qu'il existe un gradient de pression dans le sens direct ;

· en position de fermeture la fuite de sang est réduite au minimum ;

· la hauteur des clapets et le dépassement, en position d'ouverture, de ces derniers par rapport à la base, sont les moindres possibles ("low-profile valve") ;

· le passage central d'écoulement sanguin présente une section qui est plus importante que la section libre des valves existantes ;

· l'absence de zones de stagnation autorise à chaque cycle un rinçage efficace de toutes les parties de la valve et en particulier des articulations.

La présente invention a pour objet une valve cardiaque prothétique, du type comprenant :

· une pluralité de moyens d'obturation ou clapets ayant une face interne concave et étant mobiles, sous l'action des pulsations cardiaques, entre une position d'ouverture dans laquelle ils permettent l'écoulement sanguin dans un sens, dit sens direct, et une position de fermeture dans laquelle ils empêchent l'inversion de l'écoulement sanguin dans le sens opposé, dit sens inverse, chaque moyen d'obturation comportant deux coins latéraux, un bord d'attaque et un bord de fuite qui convergent dans ces deux coins ;

· une base de support des moyens d'obturation pourvue d'une paroi interne, délimitant un passage central pour l'écoulement sanguin, ainsi que d'un bord supérieur et d'un bord inférieur ;

· des moyens de suture par l'intermédiaire desquels la base est fixée aux tissus cardiaques et vasculaires, et

· des moyens de guidage en rotation et de retenue de chaque moyen d'obturation entre et dans lesdites deux positions d'ouverture et de fermeture, respectivement,

laquelle valve est caractérisée :

· en ce que lesdits moyens de guidage et de retenue de chaque clapet définissent un axe de rotation virtuel disposé à une distance de l'axe de la valve qui est supérieure à 75% du rayon de la base mesuré suivant l'axe de symétrie d'un clapet, tout en permettant l'écoulement entre la face externe du clapet correspondant et la paroi interne de la base et donc un rinçage efficace desdits moyens de guidage et de retenue ;

· en ce que chaque axe virtuel est situé entièrement à l'extérieur du clapet correspondant et est suffisamment excentré par rapport au centre de gravité du clapet, de telle façon que la résultante des forces de frottement agissant sur le clapet présente par rapport audit axe virtuel, lors de l'inversion de l'écoulement sanguin, un moment suffisant pour amorcer la fermeture du clapet correspondant, et

· en ce qu'en position d'ouverture les coins de chaque clapet viennent en appui –par l'intermédiaire d'une portion sensiblement comprise, dans ladite position d'ouverture, entre le bord supérieur de la base et le bord d'attaque de chaque clapet– sur la paroi interne de la base en des zones qui sont situées à une distance de l'axe virtuel de rotation correspondant qui est supérieure à 25% dudit rayon de la base mesuré suivant l'axe de symétrie d'un clapet, ce qui réduit les efforts nécessaires à limiter l'ouverture des clapets et réduit les efforts sollicitant les moyens de guidage à la rotation.

Selon un mode de réalisation préféré de la valve cardiaque prothétique conforme à l'invention, lesdits moyens de guidage en rotation et de retenue de chaque clapet sont constitués par :

· un premier ergot, qui fait saillie de la face externe du clapet correspondant vers l'extérieur et vers le haut à partir du point milieu de son bord d'attaque ;

· un moyen autour duquel s'articule ledit premier ergot et qui est solidaire de la paroi interne de la base ; et

· un deuxième et un troisième ergots qui font saillie sensiblement à partir du bord inférieur de la base et qui sont orientés vers l'axe de la base, à savoir l'axe d'écoulement, et légèrement vers le haut, lesdits deuxième et troisième ergots étant disposés chacun de part et d'autre par rapport au moyen d'articulation dudit premier ergot et venant en contact avec le bord d'attaque et la zone inférieure de la face interne du clapet correspondant, respectivement en position d'ouverture et de fermeture.

Selon une disposition préférée de ce mode de réalisation, ledit moyen d'articulation dudit premier ergot est constitué par un étrier sensiblement horizontal et de préférence sensiblement triangulaire, qui fait saillie de la paroi interne de la base et qui est disposé dans la moitié inférieure de cette paroi, à proximité du plan radial médian de la base, lequel étrier présente une largeur radiale inférieure à 25% du rayon de la base mesuré suivant l'axe de symétrie d'un clapet.

Selon une variante avantageuse de cette disposition, ledit moyen d'articulation dudit premier ergot est constitué par deux ergots sensiblement horizontaux qui font saillie de la paroi interne de la base et qui convergent l'un vers l'autre, les extrémités libres de ces ergots convergents étant légèrement espacées et éloignées de la paroi interne de la base d'une distance radiale inférieure à 25% du rayon de la base mesuré suivant l'axe de symétrie d'un clapet.

Selon un mode de réalisation préféré de la valve conforme à l'invention, l'encombrement radial dudit moyen d'articulation dudit premier ergot est de préférence compris entre 10% et 15% dudit rayon de la base mesuré suivant l'axe de symétrie d'un clapet.

Selon un autre mode de réalisation préféré de la valve conforme à l'invention, en position d'ouverture, chaque clapet est sensiblement parallèle à l'axe d'écoulement sanguin, à savoir que les faces interne et externe de chaque clapet sont effleurées par le sang sensiblement parallèlement à l'axe de la base.

Selon un mode de réalisation avantageux de la valve conforme à l'invention, la paroi interne de la base présente en section axiale un profil curviligne qui comporte une portion supérieure concave, suivant laquelle l'épaisseur de ladite paroi interne croît à partir du bord supérieur de la base, et une portion inférieure convexe suivant laquelle l'épaisseur de la paroi interne décroît vers le bord inférieur de la base, lesdites portions concave et convexe du profil axial de la paroi interne de cette base étant séparées par une ligne annulaire.

Selon un autre mode de réalisation avantageux de la valve conforme à l'invention, la ligne de séparation entre lesdites portions concave et convexe dudit profil axial de la paroi interne de la base varie, dans la zone comprise entre deux clapets contigus, suivant un profil radial qui est dépassant par rapport au profil radial de la zone médiane d'un clapet et qui est sensiblement symétrique par rapport aux bords de fuite de deux clapets contigus se touchant entre eux en position de fermeture, le bord d'attaque de chaque clapet présentant à son tour un profil s'adaptant à la ligne de séparation entre lesdites portions concave et convexe du profil de la paroi interne de la base, à savoir que le bord d'attaque de chaque clapet présente une portion centrale convexe et deux portions latérales rentrantes, ces dernières s'adaptant, en position de fermeture, audit profil radial dépassant de la paroi interne de la valve, l'adaptation en position de fermeture entre le bord d'attaque de chaque clapet et la paroi interne de la base se faisant de manière à réduire les fuites de sang au minimum.

Selon une disposition avantageuse de ce mode de réalisation, la portion de ladite ligne de séparation entre lesdites portions concave et convexe du profil axial de la paroi interne de la base, qui correspond à la portion convexe du bord d'attaque de chaque clapet, se situe à une distance du bord inférieur de la base qui est égale à environ 10% de la hauteur de cette base, tandis que le point milieu de la portion de ladite ligne de séparation à profil radial dépassant qui correspond aux deux portions concaves de deux clapets contigus, se situe à une distance du bord inférieur de la base qui est égale à environ 20% de sa hauteur, le passage entre lesdites deux portions de ladite ligne de séparation étant progressif.

Selon un autre mode de réalisation avantageux de la valve conforme à l'invention, chaque clapet présente au niveau de ses coins qui viennent au contact de la paroi interne de la base en position de fermeture, et sur sa face externe, un épaississement de renfort permettant que le contact se fasse par une surface et non par un point ou une ligne, limitant ainsi la pression de contact et donc l'usure, de même que le bruit de fonctionnement.

Selon un autre mode de réalisation préféré de la valve conforme à l'invention, chacun des clapets est pourvu de moyens d'avance à la fermeture, à savoir de moyens destinés à anticiper la fermeture des clapets sur le retour de l'écoulement sanguin en sens inverse, qui coopèrent avec les moyens d'amorce de la fermeture constitués par lesdits axes virtuels excentrés.

Selon une disposition préférée de ce mode de réalisation, lesdits moyens d'avance à la fermeture sont constitués par des masses magnétiques de polarités opposées ménagées au niveau des coins de chaque clapet, les polarités magnétiques des coins de deux clapets contigus étant opposées.

Selon une modalité avantageuse de cette disposition, lesdites masses magnétiques sont constituées par des petits aimants.

Selon une variante avantageuse de cette modalité, lesdites masses magnétiques sont constituées par un matériau magnétique incorporé lors de la fabrication de chaque clapet.

Selon un autre mode de réalisation avantageux de la valve conforme à l'invention, le nombre de clapets est égal à 2.

Selon une variante préférée de ce mode de réalisation, le nombre de clapets est supérieur à 2.

Selon une disposition avantageuse de cette variante, le nombre de clapets est de préférence égal à 3.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels :

· les figures 1 et 1a sont des vues de dessus et en perspective, respectivement, de la prothèse de valve cardiaque selon l'invention en position de fermeture ;

· les figures 2 et 2a sont des vues également de dessus et en perspective, respectivement, de la valve représentée aux figures 1 et 1a mais en position partiellement ouverte ;

· les figures 3 et 3a sont des vues encore également de dessus et en perspective, respectivement, de la valve précédente en position d'ouverture complète ; les figures en perspective 1a, 2a, 3a étant représentées avec des arrachements partiels ;

    · la figure 4 est une vue en coupe suivant le plan IV de la figure 1 ;

    · la figure 5 est une vue en coupe suivant le plan V de la figure 3 ;

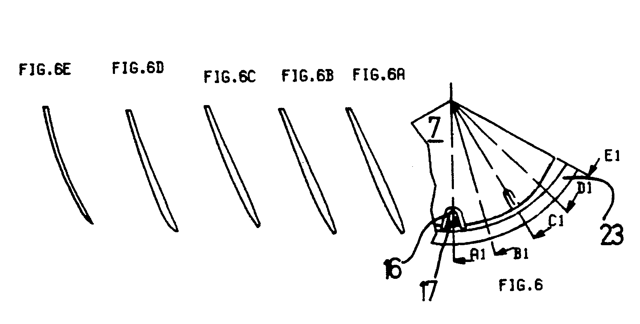

    · les figures 6a à 6e sont des vues en coupe d'un clapet de la valve selon l'invention qui montrent comment varie le profil de chaque clapet en allant de l'axe du clapet vers l'un ou l'autre de ses coins et dans le sens radial, tel que défini par les plans de coupe $A_1$, $B_1$, $C_1$, $D_1$ et $E_1$ de la figure 6 ;

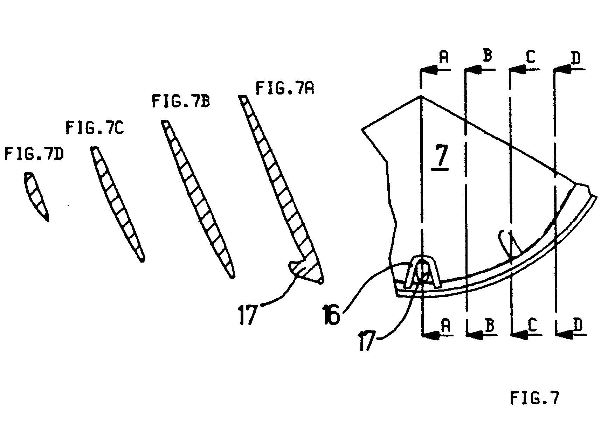

    · les figures 7a à 7d sont des vues en coupe analogues aux figures 6a à 6e qui montrent comment varie le profil de chaque clapet en allant de l'axe du clapet vers l'un ou l'autre de ses coins et parallèlement à cet axe, tel que défini par les plans de coupe $A_2$, $B_2$, $C_2$ et $D_2$ ; et

    · la figure 8 montre, avec arrachements partiels, un exemple de la valve cardiaque prothétique selon l'invention ayant une base elliptique.

Il doit être bien entendu, toutefois, que ces dessins et les parties descriptives correspondantes, sont donnés uniquement à titre d'illustration de l'objet de l'invention, dont ils ne constituent en aucune manière une limitation.

La prothèse de valve cardiaque 1 comporte une base annulaire 2 pourvue d'un passage 3 d'écoulement du sang sous l'action des pulsations cardiaques, ce passage étant délimité par une paroi interne 4 dont la configuration précise sera décrite dans ce qui va suivre.

Cette base 2 comporte également deux collerettes annulaires 23 et 24 faisant saillie de ses bords inférieur 28a et supérieur 28b et délimitant une gorge destinée à recevoir des moyens de suture (non représentés), connus en soi, pour la fixation de la valve aux tissus cardiaques et vasculaires.

L'écoulement sanguin est contrôlé par trois moyens d'obturation 5, 6 et 7 constitués par des clapets délimités par un bord d'attaque 8 et un bord de fuite 9. Chaque clapet présente une concavité interne définie par une partie centrale 10 sensiblement plane et deux parties latérales (ou coins) arquées 11 et 12. Dans ce cas, le bord de fuite 9 se présente sous la forme d'un V inversé à bras curvilignes 13 et 14 en sorte que les clapets présentent une forme sensiblement triangulaire avec un sommet 15. Il est évident que cette condition est vérifiée à partir d'un nombre de clapets égal ou supérieur à 3, tandis que dans le cas de deux clapets le bord de fuite serait, lorsqu'on les considère en position d'ouverture totale, horizontal et curviligne suivant la concavité de chaque clapet.

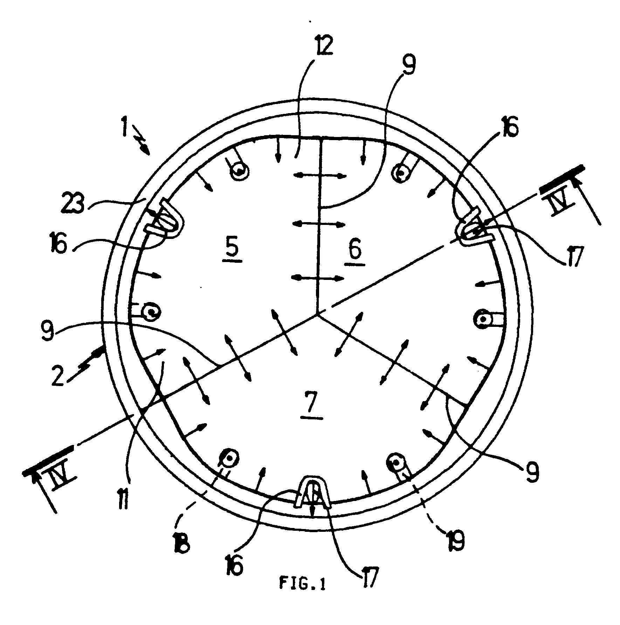

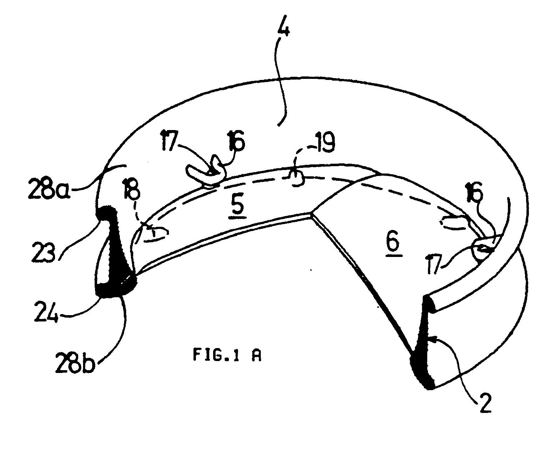

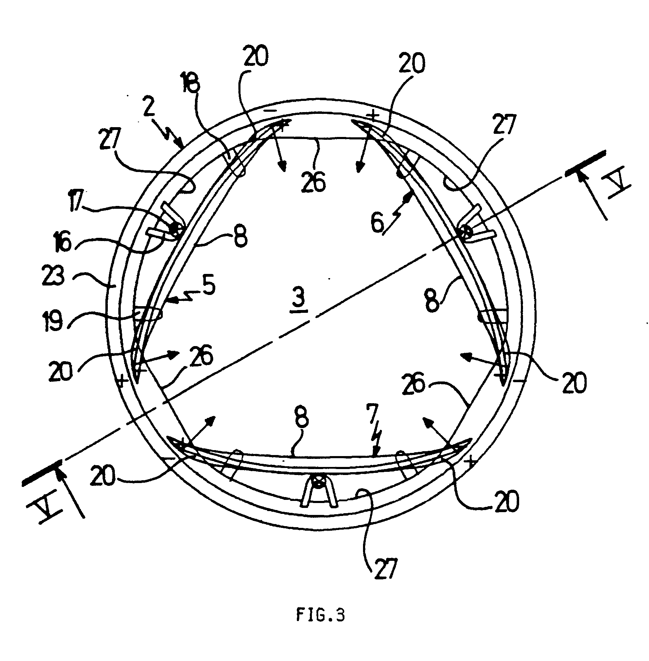

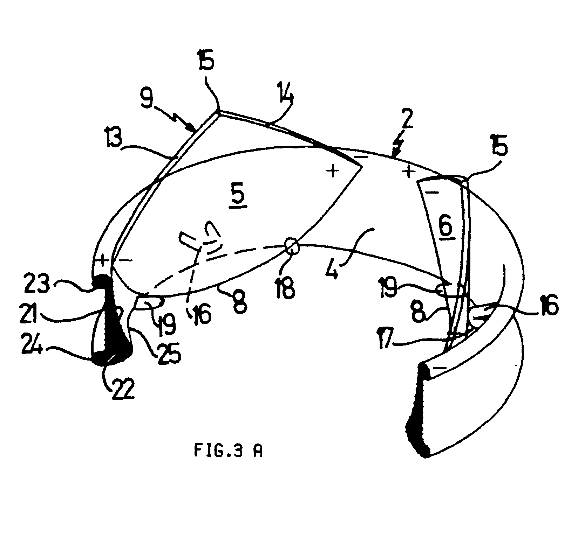

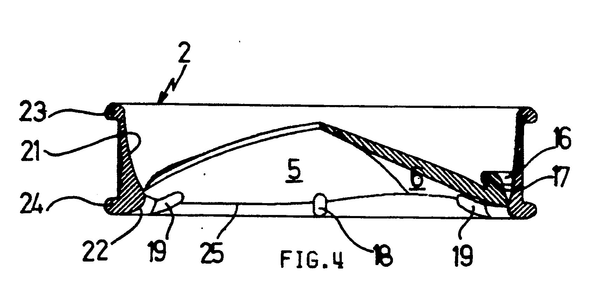

Les trois clapets 5, 6 et 7 sont mobiles entre une position d'ouverture (cf. les figures 3, 3ª et 5), dans laquelle ils permettent l'écoulement sanguin dans un sens, dit sens direct, et une position de fermeture (cf. les figures 1, 1a et 4) dans laquelle ils empêchent l'inversion de l'écoulement sanguin dans le sens opposé, dit sens inverse.

Le mouvement des trois clapets a lieu sous l'action des pulsations cardiaques grâce à la présence de moyens de guidage en rotation et de retenue.

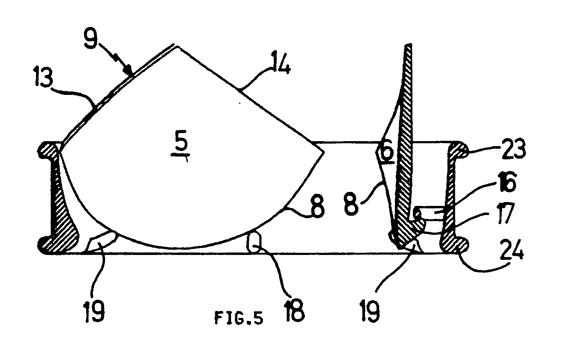

Or, conformément à l'invention, ces moyens de guidage et de retenue sont constitués par un étrier 16 sensiblement horizontal et triangulaire qui fait saillie de la paroi interne 4 de la base 2 et qui est disposé dans la moitié inférieure de cette paroi à proximité du plan radial médian de la base (cf. en particulier la figure 5), et par trois ergots.

Un premier ergot 17 fait saillie de la face externe d'un clapet vers l'extérieur et vers le haut (cf. encore en particulier la figure 5) à partir du point milieu du bord d'attaque ; cet ergot 17 coopère avec ledit étrier 16 qui est ainsi disposé du côté de la face externe du clapet.

Les deuxième et troisième ergots 18 et 19 font saillie du bord inférieur de la base 2 et sont orientés vers l'axe de la valve (on entend par axe de la valve, l'axe du cylindre de révolution de plus grand rayon que l'on peut inscrire à l'intérieur de la base) et légèrement vers le haut, ces deux ergots 18 et 19 étant disposés chacun de part et d'autre par rapport audit étrier 16, et donc audit premier ergot 17, et ils viennent en contact avec le bord d'attaque du clapet correspondant en position d'ouverture.

Il va de soi que la disposition relative entre ledit étrier 16 et lesdits ergots 17, 18 et 19 peut être inversée sans que le fonctionnement de la valve soit compromis, toutefois la disposition représentée est celle qu'on préfère dans le cadre de la présente invention.

En outre, il est aussi évident que l'étrier 16 peut être aussi rectangulaire, de même que -tout en conservant la configuration sensiblement triangulaire il peut être avantageux de le réaliser ouvert au niveau de son sommet, dans ce cas pouvant être constitué par deux ergots convergents (non représentés), sensiblement horizontaux, dont les extrémités libres sont légèrement espacées.

Dans chaque cas, l'étrier 16 et les ergots 17, 18 et 19 sont dimensionnés et positionnés de manière qu'en position d'ouverture chacun des trois clapets 5, 6 et 7 est disposé au voisinage de la paroi interne 4 de la base 2, l'encombrement radial de l'étrier étant inférieur à 25% du rayon de la base et, de préférence, compris entre 10% et 15% de ce rayon, ce qui assure une section de passage du sang qui n'a jamais été égalée dans les valves de l'Art antérieur.

On voit donc que la valve selon l'invention comporte des moyens de guidage et de retenue de chaque clapet qui définissent un axe de rotation virtuel disposé à une distance de l'axe de la valve qui est supérieure à 75% du rayon de la base 2 mesuré suivant l'axe de symétrie d'un clapet, et ce tout en permettant l'écoulement entre la face externe du clapet correspondant et la paroi interne de la base et donc un rinçage efficace des moyens de guidage et de retenue.

En outre, on peut remarquer que chaque axe virtuel est situé entièrement à l'extérieur du clapet correspondant et est suffisamment excentré par rapport au centre de gravité du clapet, de telle façon que la résultante des forces de frottement agissant sur le clapet présente par rapport audit axe virtuel, lors de l'inversion de l'écoulement sanguin, un moment suffisant pour amorcer la fermeture du clapet correspondant : c'est cette disposition excentrée des axes de rotation des clapets qui permet de placer les clapets, en position d'ouverture, sensiblement parallèlement à l'axe de l'écoulement sanguin, car les seules forces de frottement sont suffisantes pour amorcer leur fermeture et, donc, les forces de pression ne sont pas nécessaires à cet effet. (A ce propos, il y a lieu de noter que, dans l'Art antérieur, la fermeture des clapets se produit sous l'effet des seules forces de pression).

En outre, la disposition des moyens de guidage et de retenue est particulièrement avantageuse parce que les clapets sont disposés très près de la paroi interne de la base, à savoir qu'ils sont situés dans une zone où l'écoulement est ralenti par l'effet de proximité de la paroi, ce qui diminue considérablement les pertes des charges dues à la présence des clapets. De plus, l'inclinaison des ergots 18 et 19 est telle que, en position d'ouverture, la face interne des clapets est sensiblement parallèle à l'axe de la valve (axe de la base), avec la ligne joignant les deux coins 11 et 12 d'un clapet sensiblement perpendiculaire à cet axe. Le parallélisme entre l'axe de la valve (et donc le sens d'écoulement direct du sang) et chaque clapet en position d'ouverture, conjointement avec leur disposition sensiblement périphérique, élimine les problèmes de turbulence posés dans les valves antérieurement connues dus à l'inclinaison des clapets par rapport à l'axe de l'écoulement sanguin.

Or, en position d'ouverture, les coins 11 et 12 de chaque clapet viennent en appui sur la paroi interne de la base sur des zones qui sont situées à une distance de l'axe virtuel de rotation correspondant qui est supérieure à 25% du rayon de la base (toujours mesuré suivant l'axe de symétrie d'un clapet) : de cette manière, on réduit les efforts nécessaires à limiter l'ouverture des clapets et les efforts sollicitant les moyens de guidage à la rotation et de retenue de ces clapets.

En position de fermeture, on peut remarquer (cf. la figure 1) que chaque bord d'attaque comporte une portion centrale convexe 8a et deux portions latérales concaves 8b disposées au niveau des coins 11 et 12 de chaque clapet et s'adaptant au profil de la paroi interne de la base. Le profil de cette paroi comporte, en section axiale, une portion supérieure concave 21 d'épaisseur croissant et une portion inférieure convexe 22 à épaisseur décroissant. La ligne de séparation 25 entre ces deux portions 21 et 22 peut avantageusement présenter, entre deux clapets contigus, un profil radial convexe et sensiblement symétrique 26 compris entre deux portions en retrait 27.

Le profil de la paroi interne de la base et du bord d'attaque, qui s'y adapte, assure la retenue des clapets en position fermée, leur adaptation étant telle que les fuites de sang sont minimisées.

Les figures 1 et 3 montrent la répartition des efforts de retenue dans les deux positions de fermeture et d'ouverture, respectivement, ce qui est schématiquement illustré par des flèches et des petits cercles contenant un point ou une croix. Or, étant donné que les efforts de retenue les plus importants se manifestent en position fermée, il est avantageux, pour limiter l'usure, de les répartir de manière uniforme, ce qui est obtenu par le contact des clapets entre eux, suivant leurs bords de fuite, et avec la paroi interne de la base suivant leurs bords d'attaque.

C'est dans le but de limiter les contraintes locales, et donc l'usure, également en position d'ouverture,que les clapets 5, 6 et 7 présentent sur la face externe un épaississement 20 des coins 11 et 12 (cf. la figure 3) destiné à renforcer cette zone d'appui sur la base et à augmenter la surface d'appui de manière à réduire les pressions de butée et, en même temps, le bruit de fonctionnement.

Les dispositions précédentes permettent donc de répartir les efforts de fonctionnement les plus importants sur les parties massives de la valve (base et clapets) dont l'usure est faible.

La valve conforme à la présente invention est pourvue de moyens d'avance à la fermeture, à savoir de moyens destinés à anticiper la fermeture des clapets sur le retour de l'écoulement sanguin en sens inverse, et à minimiser ainsi les problèmes de régurgitation, lesquels moyens coopèrent avec les moyens d'amorce de la fermeture qui sont définis par la disposition excentrée de l'axe virtuel de rotation de chaque clapet par rapport au clapet correspondant.

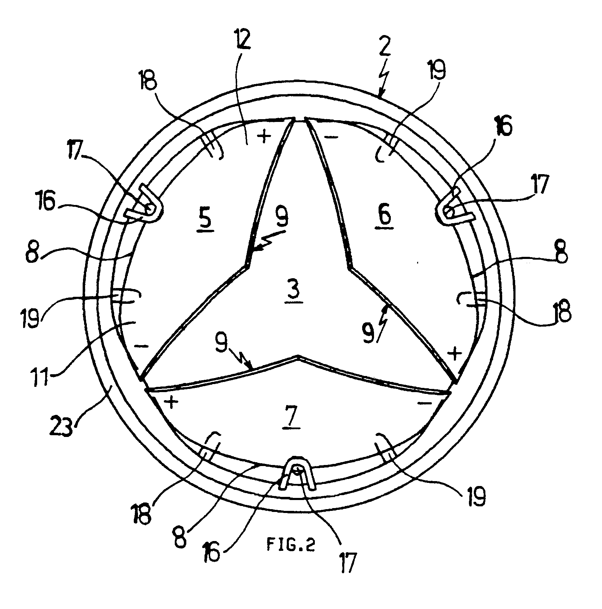

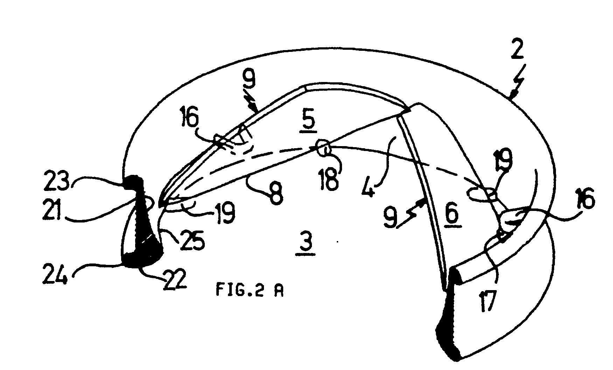

De façon préférentielle, ces moyens d'avance à la fermeture sont constitués par des masses magnétiques incorporées dans chaque clapet de manière que les coins de deux clapets contigus présentent des polarités magnétiques de signes opposés, tel que représenté schématiquement à la figure 2 par les signes − et + qui correspondent essentiellement à l'emplacement des polarités magnétiques qui interagissent entre elles.

Ces masses magnétiques ménagées dans les coins de chaque clapet peuvent coopérer aussi avec des masses magnétiques supplémentaires qui sont disposées dans la base entre deux clapets contigus et dont l'encombrement est sensiblement égal à la distance qui existe entre les coins adjacents de deux clapets contigus en position d'ouverture, chaque masse magnétique supplémentaire étant disposée dans la base de manière que ses polarités correspondent aux polarités de signe opposé des masses magnétiques ménagées dans les coins des clapets.

La disposition des masses magnétiques supplémentaires est illustrée schématiquement aux figures 3 et 3a en indiquant essentiellement l'emplacement des polarités + et − de chacune.

Les masses magnétiques sont calculées pour que l'attraction entre les coins de clapets voisins, visant à fermer la valve, s'oppose exactement aux forces de pression et de frottement qui tendent à l'ouvrir lorsque la vitesse de l'écoulement en sens direct atteint un certain pourcentage de sa valeur maximale. Ainsi la fermeture de la valve s'annoncera avant le retour de l'écoulement.

Les masses magnétiques peuvent être constituées, de façon connue en soi, par des petits aimants, fixés sur les coins des clapets par tout moyen approprié, ou par un matériau magnétique incorporé lors de leur fabrication sous forme finement dispersée.

En ce qui concerne les matériaux susceptibles d'être utilisés pour la construction de la prothèse de valve cardiaque, le choix est orienté vers le groupe des matériaux biocompatibles, tels que, de préférence, le matériau connu sous le nom de marque de fabrique "PYROLYTE" en raison de son inertie, de sa résistance à l'usure et de sa propriété de thromborésistance (désormais reconnues) dans le milieu physiologique, ainsi que le titane et les matériaux connus sous leurs noms de marques de fabrique suivants : "DERLIN", "BIOLITE", "STELLITE" et d'autres encore connus des techniciens en la matière.

Lorsqu'on utilise le carbone pyrolytique, à cause des impératifs mécaniques connus qui empêchent de réaliser la totalité de la prothèse en un tel matériau, on utilise celui-ci comme un revêtement déposé sur un substrat selon une technique également connue en elle-même (cf. le brevet américain n° 3 526 005), le substrat étant réalisé suivant la forme désirée et étant constitué de façon typique par du graphite : de toutes façons, le choix du substrat et de l'épaisseur du revêtement de carbone pyrolytique les plus appropriés entrent dans la compétence d'un technicien en la matière.

En outre, en ce qui concerne en particulier la réalisation de la base de support des moyens d'obturation, il est préférable de la réaliser en une seule pièce évitant, dans la mesure du possible, l'emploi d'une base formée par assemblage de plusieurs éléments, car cela entraîne les risques potentiels de séparation des éléments composants et de formation de zones de stagnation du sang.

La réalisation en une seule pièce est aussi envisageable, sans craindre les problèmes de montage, parce qu'on peut utiliser la propriété du carbone pyrolytique d'être déformable élastiquement de façon suffisante pour permettre la mise en place des moyens d'obturation de la valve conforme à la présente invention, si sa base et/ou ses clapets sont réalisés en carbone pyrolytique.

Ci-après sont reprises certaines parties de la description précédente pour les mettre en rapport avec le fonctionnement de la valve.

En position fermée, celle-ci s'oppose à l'écoulement en sens inverse par la position des clapets qui assurent l'étanchéité entre ces clapets, le long de leurs bords de fuite, et un minimum de fuites de sang entre leur bord d'attaque et la base de la valve.

Dans cette position, les efforts de pression sur les moyens d'obturation sont repris :

· par la base orientée vers l'aval de l'écoulement en sens direct, le long du bord d'attaque des moyens d'obturation ;

· par les moyens de retenue situés entre chaque moyen d'obturation et la base.

Ces différents points d'appui, étant bien répartis autour de chaque moyen d'obturation, assurent que les contraintes ne sont pas amplifiées par effet de levier, comme c'est le cas quand seule l'articulation reprend les efforts.

Dès qu'il existe un gradient de pression dans le sens direct, l'ouverture des moyens d'obturation se fait naturellement, étant donné que leur axe de rotation est très excentré par rapport au point d'application des forces de pression.

Ce mouvement est arrêté lorsque les coins des moyens d'obturation arrivent en appui sur la base. Le choc des moyens d'obturation sur la base se fait entre 2 parties massives et sur une surface importante, ce qui a pour conséquence de :

· minimiser les contraintes dans les matériaux ;

· diminuer le bruit de fonctionnement.

Les moyens de retenue de chaque moyen d'obturation empêchent celui-ci de s'échapper dans le sens du flot. Les efforts nécessaires à cette fonction sont faibles, car ils ne sont produits que par les forces de frottement de l'écoulement sur les parois des obturateurs (les forces de pression sont très faibles car les obturateurs sont parallèles à l'écoulement).

En position d'ouverture, les moyens d'obturation sont placés au voisinage des parois et parallèlement à l'écoulement, ce qui a pour effet :

· de les placer dans une partie où l'écoulement est ralenti par la proximité des parois (effets de viscosité), donc de minimiser les pertes de charge par frottement fluide ;

· d'éviter la création de tourbillons au voisinage des moyens d'obturation, car ceux-ci ne subissent pas ou peu d'effort de pression, étant parallèles à l'écoulement ;

· de laisser libre de tout obstacle une partie centrale de l'écoulement qui représente plus de 80% de la section totale de passage de la base dans les réalisations préférées de l'invention.

Dès que le sens de l'écoulement s'inverse, les forces de frottement qui s'exercent sur les parois des moyens d'obturation exercent un moment par rapport à leur axe qui tend à amorcer le mouvement de fermeture. Aussitôt que le moyen d'obturation a pris un peu d'incidence par rapport à l'écoulement inverse, des forces de pression s'exercent sur lui qui provoquent un basculement très rapide et la fermeture de la valve.

Si la valve est équipée du dispositif introduisant une avance à la fermeture, tel que décrit précédemment, l'amorce de fermeture due aux forces de frottement bénéficie d'une amorce de fermeture additionnelle qui est obtenue par l'attraction magnétique des moyens d'obturation et se produit dès que la vitesse de l'écoulement direct passe en dessous d'une certaine valeur, donc avant l'inversion de l'écoulement. Dans un cas extrême, on peut faire en sorte que la fermeture se fasse totalement par les forces magnétiques et soit complète au moment de l'inversion de l'écoulement. Dans ce cas, le taux de régurgitation serait nul. En réalité, ce cas n'est probablement pas optimal pour le rendement global de la valve, le meilleur rendement étant lié à un taux d'aimantation des moyens d'obturation qui peut être facilement déterminé par un technicien en la matière.

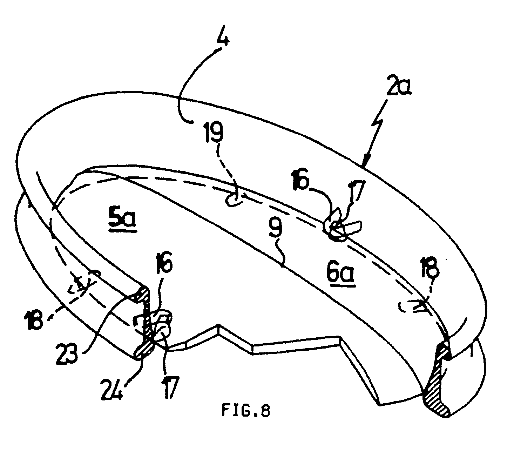

Il va de soi que, bien que la description ait été limitée aux prothèses de valves cardiaques de forme circulaire, les dispositions qui précèdent s'appliquent également aux valves artificielles de forme elliptique ou ovale (telle que la valve illustrée à la figure 8 dont la base 2a comporte deux clapets 5a et 6a représentés en position de fermeture) ou aux valves ayant une section convexe a priori quelconque, ainsi qu'à toutes les applications dans lesquelles est nécessaire de pourvoir à un contrôle de fluide, notamment de fluides cryogéniques ou autres.

Ainsi que cela ressort de ce qui précède, l'invention ne se limite nullement à ceux de ses modes de mise en oeuvre, de réalisation et d'application qui viennent d'être décrits de façon plus explicite ; elle en embrasse, au contraire, toutes les variantes qui peuvent venir à l'esprit du technicien en la matière, sans s'écarter du cadre, ni de la portée de la présente invention : par exemple, bien que les dessins représentent des valves ayant une base circulaire ou elliptique, il va de soi que la valve cardiaque prothétique selon l'invention peut présenter une forme générale externe convexe a priori quelconque de même qu'une base dont la paroi interne peut présenter un profil approprié à la configuration adoptée pour chaque clapet. En outre, bien qu'on préfère utiliser un nombre de clapets supérieur ou égal à 2, essentiellement pour que la valve reste du type dit (dans la terminologie anglo-saxonne) "low profile", il va de soi que les moyens de guidage et de retenue conformes à la présente invention sont adaptables aussi à l'emploi d'un clapet unique dont la forme peut être spéciale, notamment assimilable à une coquille.

De plus, bien qu'on préfère utiliser un nombre de clapets égal à 2 lorsque la base présente une forme allongée dans une direction (à savoir tendant à la forme ovale ou elliptique) et un nombre de clapets égal à 3 lorsque la forme est circulaire ou approximativement telle, il va de soi que pour une forme donnée de la base, le nombre de clapets peut être a priori quelconque.

## Revendications

1. Valve cardiaque prothétique (1), du type comprenant :

· une pluralité de moyens d'obturation ou clapets (5, 6, 7) ayant une face interne concave et étant mobiles, sous l'action des pulsations cardiaques, entre une position d'ouverture dans laquelle ils permettent l'écoulement sanguin dans un sens, dit sens direct, et une position de fermeture dans laquelle ils empêchent l'inversion de l'écoulement sanguin dans le sens opposé, dit sens inverse, chaque moyen d'obturation comportant deux coins latéraux (11, 12), un bord d'attaque (8) et un bord de fuite (9) qui convergent dans lesdits deux coins latéraux (11, 12) ;

· une base (2, 2a) de support des moyens d'obturation (5, 6, 7) pourvue d'une paroi interne (4) délimitant un passage central (3) pour l'écoulement sanguin, ainsi que d'un bord supérieur (28b) et d'un bord inférieur (28a) ;

· des moyens de suture, par l'intermédiaire desquels la base est fixée aux tissus cardiaques et vasculaires, et

· des moyens de guidage en rotation et de retenue de chaque moyen d'obturation entre et dans lesdites deux positions d'ouverture et de fermeture, respectivement,

laquelle valve est caractérisée :

– en ce que lesdits moyens de guidage et de retenue de chaque clapet définissent un axe de rotation virtuel disposé à une distance de l'axe de la valve qui est supérieure à 75% du rayon de la base (2, 2a) mesuré suivant l'axe de symétrie d'un clapet, tout en permettant l'écoulement entre la face externe du clapet correspondant et la paroi interne de la base et donc un rinçage efficace desdits moyens de guidage et de retenue ;

– en ce que chaque axe virtuel est situé entièrement à l'extérieur du clapet correspondant et est suffisamment excentré par rapport au centre de gravité du clapet, de telle façon que la résultante des forces de frottement agissant sur le clapet présente par rapport audit axe virtuel, lors de l'inversion de l'écoulement sanguin, un moment suffisant pour amorcer la fermeture du clapet correspondant, et

– en ce que, en position d'ouverture, les coins latéraux (11, 12) de chaque clapet viennent en appui

–par l'intermédiaire d'une portion sensiblement comprise, dans cette position d'ouverture, entre le bord supérieur (28a) de la base et le bord d'attaque (8) de chaque clapet- sur la paroi interne de la base en des zones qui sont situées à une distance de l'axe virtuel de rotation correspondant qui est supérieure à 25% dudit rayon de la base mesuré suivant l'axe de symétrie d'un clapet, ce qui réduit d'une part les efforts nécessaires à limiter l'ouverture des clapets et, d'autre part, les efforts sollicitant les moyens de guidage à la rotation et de retenue de ces clapets.

2. Valve cardiaque prothétique selon la revendication 1, caractérisée en ce que lesdits moyens de guidage en rotation et de retenue de chaque clapet sont constitués par :

· un premier ergot (17), qui fait saillie de la face externe du clapet correspondant vers l'extérieur et vers le haut à partir du point milieu de son bord d'attaque (8) ;

· un moyen autour duquel s'articule ledit premier ergot (17) et qui est solidaire de la paroi interne (4) de la base (2, 2a) ; et

· un deuxième et un troisième ergots (18, 19) qui font saillie sensiblement à partir du bord inférieur de la base (2, 2a), et qui sont orientés vers l'axe de la base, à savoir l'axe d'écoulement, et légèrement vers le haut, lesdits deuxième et troisième ergots (18, 19) étant disposés chacun de part et d'autre par rapport au moyen d'articulation dudit premier ergot (17) et venant en contact avec le bord d'attaque (8) et la zone inférieure de la face interne du clapet correspondant, respectivement en position d'ouverture et de fermeture.

3. Valve cardiaque prothétique selon la revendication 2, caractérisée en ce que ledit moyen d'articulation dudit premier ergot est constitué par un étrier (16) sensiblement horizontal et de préférence sensiblement triangulaire, qui fait saillie de la paroi interne (4) de la base (2, 2a) et qui est disposé dans la moitié inférieure de cette paroi, à proximité du plan radial médian de la base, lequel étrier présente une largeur radiale inférieure à 25% du rayon de la base mesuré suivant l'axe de symétrie d'un clapet.

4. Valve cardiaque prothétique selon la revendication 2, caractérisée en ce que ledit moyen d'articulation dudit premier ergot est constitué par deux ergots sensiblement horizontaux qui font saillie de la paroi interne de la base et qui convergent l'un vers l'autre, les extrémités libres de ces ergots convergents étant légèrement espacées entre elles et éloignées de la paroi interne de la base d'une distance radiale inférieure à 25% du rayon de la base mesuré suivant l'axe de symétrie d'un clapet.

5. Valve cardiaque prothétique selon l'une quelconque des revendications 3 et 4, caractérisée en ce que l'encombrement radial dudit moyen d'articulation dudit premier ergot (17) est de préférence compris entre 10% et 15% dudit rayon de la base mesuré suivant l'axe de symétrie d'un clapet.

6. Valve cardiaque prothétique selon l'une quelconque des revendications 1 à 5, caractérisée en ce que, en position d'ouverture, chaque clapet (5, 6, 7) est sensiblement parallèle à l'axe d'écoulement sanguin, à savoir que les faces interne et externe de chaque clapet sont effleurées par le sang sensiblement parallèlement à l'axe de la base.

7. Valve cardiaque prothétique selon l'une quelconque des revendications 1 à 5, caractérisée en ce que la paroi interne (4) de la base présente en section axiale un profil curviligne qui comporte une portion supérieure concave (21) suivant laquelle l'épaisseur de ladite paroi interne croît à partir du bord supérieur de la base, et une portion inférieure convexe (22) suivant laquelle l'épaisseur de la paroi interne décroît vers le bord inférieur de la base, lesdites portions concave (21) et convexe (22) du profil axial de la paroi interne de cette base étant séparées par une ligne annulaire (25).

8. Valve cardiaque prothétique selon la revendication 7, caractérisée en ce que la ligne de séparation (25) entre lesdites portions concave (21) et convexe (22) dudit profil axial de la paroi interne (4) de la base varie, dans la zone comprise entre deux clapets contigus (5-6 ; 6-7 ; 7-5), suivant un profil radial qui est dépassant par rapport au profil radial de la zone médiane d'un clapet et qui est sensiblement symétrique par rapport aux bords de fuite (9) de deux clapets contigus se touchant entre eux en position de fermeture, le bord d'attaque (8) de chaque clapet présentant à son tour un profil s'adaptant à ladite ligne de séparation (25) entre lesdites portions concave et convexe (21 et 22) du profil de la paroi interne (4) de la base, à savoir que le bord d'attaque (8) de chaque clapet présente une portion centrale convexe (8a) et deux portions latérales rentrantes (8b), ces dernières s'adaptant, en position de fermeture, audit profil radial dépassant de la paroi interne de la valve, l'adaptation en position de fermeture entre le bord d'attaque de chaque clapet et la paroi interne de la base se faisant de manière à réduire les fuites de sang au minimum.

9. Valve cardiaque prothétique selon la revendication 8, caractérisée en ce que la portion (27) de ladite ligne de séparation (25) entre lesdites portions concave et convexe (21, 22) du profil axial de la paroi interne (4) de la base, qui correspond à la portion convexe (8a) du bord d'attaque (8) de chaque clapet (5, 6, 7), se situe à une distance du bord inférieur de la base qui est égale à environ 10% de la hauteur de cette base, tandis que le point milieu de la portion (26) de ladite ligne de séparation (25) à profil radial dépassant qui correspond aux deux portions concaves (8b) de deux clapets contigus (5,6-6,7-7,5) se situe à une distance du bord inférieur de la base qui est égale à environ 20% de sa hauteur, le passage entre lesdites deux portions (26, 27) de ladite ligne de séparation (25) étant progressif.

10. Valve cardiaque prothétique selon l'une quelconque des revendications 1 à 9, caractérisée en ce que chaque clapet (5, 6, 7) présente au niveau de ses coins (11, 12) qui viennent au contact de la paroi interne (4) de la base en position de fermeture, et sur sa face externe, un épaississement (20) de renfort permettant que le contact se fasse par une surface et non par un point ou une ligne, limitant ainsi la pression de contact, et donc l'usure, de même que le bruit de fonctionnement.

11. Valve cardiaque prothétique selon l'une quelconque des revendications 1 à 10, caractérisée en ce que chacun des clapets (5, 6, 7) est pourvu de moyens d'avance à la fermeture, à savoir de moyens destinés à anticiper la fermeture des clapets sur le retour de l'écoulement sanguin en sens inverse, qui coopèrent avec les moyens d'amorce de la fermeture constitués par lesdits axes virtuels excentrés.

12. Valve cardiaque prothétique selon la revendication 11, caractérisée en ce que lesdits moyens d'avance à la fermeture sont constitués par des masses magnétiques de polarités opposées ménagées au niveau des coins de chaque clapet, les polarités magnétiques des coins de deux clapets contigus étant opposés.

13. Valve cardiaque prothétique selon la revendication 12, caractérisée en ce que lesdites masses magnétiques ménagées au niveau des coins de chaque clapet coopèrent avec des masses magnétiques supplémentaires qui sont disposées dans la base entre deux clapets contigus et dont l'encombrement est sensiblement égal à la distance qui existe entre les coins adjacents de deux clapets contigus en position d'ouverture, chaque masse magnétique supplémentaire étant disposée dans la base de manière que ses polarités correspondent aux polarités de signe opposé des masses magnétiques ménagées dans les coins des clapets.

14. Valve cardiaque prothétique selon l'une quelconque des revendications 12 et 13, caractérisée en ce que lesdites masses magnétiques sont constituées par des petits aimants.

15. Valve cardiaque prothétique selon l'une quelconque des revendications 12 et 13, caractérisée en ce que lesdites masses magnétiques sont constituées par un matériau magnétique incorporé lors de la fabrication de chaque clapet ou de la base.

16. Valve cardiaque prothétique selon l'une quelconque des revendications 1 à 15, caractérisée en ce que la base (2) est circulaire ou sensiblement circulaire.

17. Valve cardiaque prothétique selon l'une quelconque des revendications 1 à 15, caractérisée en ce que la base (2a) est elliptique ou sensiblement elliptique.

18. Valve cardiaque prothétique selon l'une quelconque des revendications 1 à 17, caractérisée en ce que le nombre de clapets (5a, 6a) est égal à 2.

19. Valve cardiaque prothétique selon l'une quelconque des revendications 1 à 17, caractérisée en ce que le nombre de clapets (5,6,7) est supérieur à 2.

20. Valve cardiaque prothétique selon la revendication 19, caractérisée en ce que le nombre de clapets (5,6,7) est égal à 3.

## Ansprüche

1. Herzklappenprothese (1) der Art, die aufweist: eine Mehrzahl von Verschlußmitteln oder von Verschlußklappen (5, 6, 7), die eine konkave Innenfläche besitzen und unter der Wirkung der Herzpulsschläge zwischen einer Offenstellung in der sie die Blutströmung in eine Flußrichtung genannte Richtung ermöglichen und einer Schließstellung, in der sie den Umkehr der Blutströmung in die Gegenrichtung genannte entgegengesetzte Richtung verhindern, beweglich sind, wobei jedes Verschlußmittel zwei seitliche Ecken (11, 12), eine Vorderkante (8) und eine Hinterkante (9) aufweist, die zu den zwei seitlichen Ecken (11, 12) konvergieren,
eine Trägerbasis (2, 2a) der Verschlußmittel (5, 6, 7), die mit einer einen zentralen Durchlaß (3) für die Blutströmung abgrenzenden Innenwand (4) sowie mit einem Oberrand (28b) und einem Unterrand (28a) versehen ist,
Nahtmittel, über welche die Basis an den Herz- und Gefäßgeweben befestigt ist und
Drehführungs- und Zurückhaltemittel jedes Verschlußmittels zwischen und in den zwei Offen- bzw. Schließstellungen, wobei die Klappe dadurch gekennzeichnet ist,
daß die Führungs- und Zurückhaltemittel jeder Verschlußklappe eine virtuelle Drehachse definieren, die in einem Abstand von der Achse der Klappe angeordnet ist, der größer als 75% des längs der Symmetrieachse einer Verschlußklappe gemessenen Radius der Basis (2, 2a) ist, wodurch die Strömung zwischen der Außenfläche der entsprechenden Verschlußklappe und der Innenwand der Basis und somit eine wirksame Spülung der Führungs- und Zurückhaltemittel ermöglicht ist, daß jede virtuelle Achse ganz außerhalb der entsprechenden Verschlußklappe und bezüglich des Schwerpunktes der Verschlußklappe hinreichend exzentrisch so liegt, daß die Resultierende der auf die Verschlußklappen wirkenden Reibungskräfte zur Zeit der Umkehr der Blutströmung bezüglich der virtuellen Achse ein hinreichendes Moment ausübt, um das Schließen der entsprechenden Verschlußklappe einzuleiten und
daß sich die seitlichen Ecken (11, 12) jeder Verschlußklappe in Offenstellung über einen in dieser Offenstellung im wesentlichen zwischen dem Oberrand (28a) der Basis und der Vorderkante (8) jeder Verschlußklappe enthaltenen Teil auf die Innenwand der Basis in Bereichen abstützen, die in einem Abstand von der entsprechenden virtuellen Drehachse liegen, der größer als 25% des längs der Symmetrieachse einer Verschlußklappe gemessenen Radius der Basis ist, wodurch einerseits die notwendigen Kräfte zum Begrenzen der Öffnung der Verschlußklappen und andererseits die Kräfte verringert werden, welche die Drehführungs- und Zurückhaltemittel dieser Verschlußklappen beanspruchen.

2. Herzklappenprothese nach Anspruch 1, dadurch gekennzeichnet, daß die Drehführungs- und Zurückhaltemittel jeder Verschlußklappe aus folgendem bestehen :
einer ersten Nase (17), die von der Außenfläche der entsprechenden Verschlußklappe nach außen und nach oben von dem Mittelpunkt ihrer Vorderkante (8) an einen Vorsprung bildet,
einem Mittel, um das sich die erste Nase (17) einfügt und das mit der Innenwand (4) der Basis (2, 2a) verbunden ist und einer zweiten und einer dritten Nase (18, 19), die im wesentlichen von dem Unterrand der Basis (2, 2a) an einen Vorsprung bildet und die gegen die Achse der Basis, nämlich die Strömungsachse ; und ein wenig nach oben ausgerichtet sind, wobei jede der zweiten und dritten Nasen (18, 19) zu beiden Seiten bezüglich des Gelenkmittels der ersten Nase (17) angeordnet ist und mit der Vorderkante (8) bzw. dem Unterbereich der Innenfläche der entsprechenden Verschlußklappe in Offen- und Schließstellung in Berührung kommt.

3. Herzklappenprothese nach Anspruch 2, dadurch gekennzeichnet, daß das Gelenkmittel der ersten Nase aus einem Bügel (16) besteht, der im wesentlichen horizontal und bevorzugt im wesentlichen dreieckig ist, der von der Innenwand (4) der Basis (2, 2a) einen Vorsprung bildet und der in der unteren Hälfte dieser Wand, in der Nähe der Radialmittelebene der Basis angeordnet ist, wobei der Bügel eine Radialbreite aufweist, die kleiner als 25% des längs der Symmetrieachse einer Verschlußklappe gemessenen Radius der Basis ist.

4. Herzklappenprothese nach Anspruch 2, dadurch gekennzeichnet, daß das Gelenkmittel der ersten Nase aus zwei im wesentlichen horizontalen Nasen besteht,die von der Innenwand der Basis einen Vorsprung bilden und die gegeneinander konvergieren, wobei die freien Enden dieser konvergenten Nasen zueinander ein wenig beabstandet sind und sich von der Innenwand der Basis um einen Radialabstand entfernt befinden, der kleiner als 25% des längs der Symmetrieachse einer Verschlußklappe gemessenen Radius der Basis ist.

5. Herzklappenprothese nach irgend einem der Ansprüche 3 und 4, dadurch gekennzeichnet, daß die Radialabmessung des Gelenkmittels der ersten Nase (17) bevorzugt zwischen 10% und 15% des längs der Symmetrieachse einer Verschlußklappe gemessenen Radius liegt.

6. Herzklappenprothese nach irgend einem der Ansprüche 1 bis 5, dadurch gekennzeichnet, daß jede Verschlußklappe (5, 6, 7) in einer Offenstellung im wesentlichen parallel zu der Blutströmungsachse liegt, das heißt, daß die Innen- und Außenflächen jeder Verschlußklappe im wesentlichen parallel zu der Achse der Basis von dem Blut berührt werden.

7. Herzklappenprothese nach irgend einem der Ansprüche 1 bis 5, dadurch gekennzeichnet, daß die Innenwand (4) der Basis im Achsschnitt ein krummliniges Profil aufweist, das umfaßt : einen konkaven Oberabschnitt (21), längs dessen die Dicke der Innenwand von dem Oberrand der Basis an zunimmt und einen konvexen Unterabschnitt (22), längs dessen die Dicke der Innenwand gegen den Unterrand der Basis abnimmt, wobei die konkaven (21) und konvexen (22) Abschnitte des Achsschnittprofiles der Innenwand dieser Basis durch eine ringförmige Linie (25) getrennt sind.

8. Herzklappenprothese nach Anspruch 7, dadurch gekennzeichnet, daß die Trennlinie (25) zwischen den konkaven (21) und konvexen (22) Abschnitten des Achsschnittprofiles der Innenwand (4) der Basis in dem zwischen zwei angrenzenden Verschlußklappen (5-6 ; 6-7 ; 7-5) eingeschlossenen Bereich längs eines Radialprofiles variiert, das bezüglich des Radialprofiles des Mittelbereiches einer Verschlußklappe herausragend ist und das bezüglich der Hinterkanten (9) zweier angrenzender Verschlußklappen im wesentlichen symmetrisch ist, die sich einander in Schließstellung berühren, wobei die Vorderkante (8) jeder Verschlußklappe ihrerseits ein Profil aufweist, das sich an die Trennlinie (25) zwischen den konkaven und konvexen Abschnitten (21 und 22) des Profiles der Innenwand (4) der Basis anschmiegt, das heißt, daß die Vorderkante (8) jeder Verschlußklappe einen zentralen kovexen Abschnitt (8a) und zwei seitliche einspringende Abschnitte (8b) aufweist, wobei sich diese letzteren in Schließstellung an das herausragende Radialprofil der Innenwand der Klappe anschmiegen und das Anschmiegen in Schließstellung zwischen der Vorderkante jeder Verschlußklappe und der Innenwand der Basis erfolgt, um die die Leckströmung des Blutes auf ein Minimum zu verringern.

9. Herzklappenprothese nach Anspruch 8, dadurch gekennzeichnet, daß der Abschnitt (27) der Trennlinie (25) zwischen den konkaven und konvexen Abschnitten (21, 22) des Achsschnittprofiles der Innenwand (4) der Basis, welcher dem konvexen Abschnitt (8a) der Vorderkante (8) jeder Verschlußklappe (5, 6, 7) entspricht, in einem-Abstand von dem Unterrand der Basis liegt, der ungefähr 10% der Höhe dieser Basis gleich ist, wogegen der Mittelpunkt des Abschnittes (26) der Trennlinie (25) mit einem herausragenden Radialprofil, das den zwei konkaven Abschnitten (8b) der zwei angrenzenden Verschlußklappen (5, 6-6, 7-7, 5) entspricht, in einem Abstand von dem Unterrand der Basis liegt, der ungefähr gleich 20% ihrer Höhe ist, wobei der Obergang zwischen den zwei Abschnitten (26, 27) der Trennlinie (25) progressiv ist.

10. Herzklappenprothese nach irgend einem der Ansprüche 1 bis 9, dadurch gekennzeichnet, daß jede Verschlußklappe (5, 6, 7) auf der Höhe ihrer Ecken (11, 12), die mit der Innenwand (4) der Basis in Schließstellung in Berührung kommen, und auf ihrer Außenfläche eine Verstärkungsverdickung (20) aufweist, wodurch es möglich ist, daß die Berührung über eine Oberfläche und nicht über einen Punkt oder eine Linie erfolgt, wodurch der Berührungsdruck und die Abnutzung ebenso wie das Betriebsgeräusch begrenzt wird.

11. Herzklappenprothese nach irgend einem Anspruch 1 bis 10, dadurch gekennzeichnet, daß jede der Verschlußklappen (5, 6, 7) mit Vortreibmitteln zum Verschließen versehen ist, nämlich mit Mitteln, die zum Vorwegnehmen des Verschließens der Verschlußklappen bei Rückkehr der Blutströmung in Gegenrichtung bestimmt sind, die mit den Einleitmitteln des Verschließens zusammenwirken, welche aus den exzentrierten virtuellen Achsen bestehen.

12. Herzklappenprothese nach Anspruch 11, dadurch gekennzeichnet, daß die Vortreibmittel zum Verschließen aus magnetischen Massen entgegengesetzter Polungen bestehen, die auf der Höhe der Ecken jeder Verschlußklappe angebracht sind, wobei die magnetischen Polungen der Ecken der zwei angrenzenden Verschlußklappen entgegengesetzt sind.

13. Herzklappenprothese nach Anspruch 12, dadurch gekennzeichnet, daß die-auf der Höhe der Ecken jeder Verschlußklappe angebrächten magnetischen Massen mit zusätzlichen magnetischen Massen zusammenwirken, die in der Basis zwischen zwei angrenzenden Verschlußklappen angeordnet sind und deren Abmessung im wesentlichen gleich dem Abstand ist, der zwischen den anliegenden Ecken der zwei angrenzenden Verschlußklappen in Offenstellung vorhanden ist, wobei jede zusätzliche magnetische Masse in der Basis so angeordnet ist, daß ihre Polungen den Polungen entgegengesetzten Vorzeichens der magnetischen Massen entsprechen, die in den Ecken der Verschlußklappen angebracht sind.

14. Herzklappenprothese nach irgend einem der Ansprüche 12 und 13, dadurch gekennzeichnet, daß die magnetischen Massen aus kleinen Magneten bestehen.

15. Herzklappenprothese nach irgend einem der Ansprüche 12 und 13, dadurch gekennzeichnet, daß die magnetischen Massen aus einem magnetischen Material bestehen, das bei der Herstellung jeder Verschlußklappe oder der Basis eingearbeitet wird.

16. Herzklappenprothese nach irgend einem der Ansprüche 1 bis 15, dadurch gekennzeichnet, daß die Basis (2) kreisförmig oder im wesentlichen kreisförmig ist.

17. Herzklappenprothese nach irgend einem der Ansprüche 1 bis 15, dadurch gekennzeichnet, daß die Basis (2a) elliptisch oder im wesentlichen elliptisch ist.

18. Herzklappenprothese nach irgend einem der Ansprüche 1 bis 17, dadurch gekennzeichnet, daß die Anzahl der Verschlußklappen (5a, 6a) gleich 2 ist.

19. Herzklappenprothese nach irgend einem der Ansprüche 1 bis 17, dadurch gekennzeichnet, daß die Anzahl der Verschlußklappen (5, 6, 7) größer als 2 ist.

20. Herzklappenprothese nach Anspruch 19, dadurch gekennzeichnet, daß die Anzahl der Verschlußklappen (5, 6, 7) gleich 3 ist.

## Claims

1. Prosthetic heart valve (1) of the type comprising :

· a plurality of closure means or flap valves (5, 6, 7) having an internal concave face and being movable, under the action of the heart pulsations, between an open position in which they allow blood flow in one direction, called forward direction, and a closed position in which they prevent reversal of the blood flow in the opposite direction, called reverse direction, each closure means comprising two lateral corners (11, 12), a leading edge (8) and a trailing edge (9) which converge in said two lateral corners (11, 12) ;

· a base (2, 2a) supporting the closure means (5, 6, 7) and having an internal wall (4) defining a central passage (3) for the blood flow, as well as an upper edge (28b) and a lower edge (28a) ;

· suture means, by means of which the base is fixed to the heart and vascular tissues ; and

· means for guiding in rotation and retaining each closure means between and in said two open and closed positions, respectively, which valve is characterized :

– in that said means for guiding and retaining each flap valve define a virtual axis of rotation disposed at a distance from the axis of the valve which is greater than 75% of the radius of the base (2, 2a) measured along the axis of symmetry of a flap valve, while permitting the flow between the external face of the corresponding flap valve and the internal face of the base and so efficient rinsing of said guide and retention means ;

– in that each virtual axis is situated entirely outside the corresponding flap valve and is sufficiently off-centred with respect to the centre of gravity of the flap valve, so that the resultant of the friction forces acting on the flap valve has, with respect to said virtual axis during reversal of the blood flow, a sufficient moment to begin closure of the corresponding flap valve, and

– in that, in the open-position, the lateral corners (11, 12) of each flap valve bear - via a portion substantially located, in this open position, between the upper edge (28a) of the base and the leading edge (8) of each flap valve - on the internal wall of the base in zones which are situated at a distance from the corresponding virtual axis of rotation which is greater than 25% of the radius of the base measured along the axis of symmetry of a flap valve, which reduces on the one hand the force required for limiting opening of the flap valves and, on the other, the forces urging the rotation guide and retention means of these flap valves.

2. Prosthetic heart valve according to claim I, characterized in that said rotation guide and retention means for each flap valve are formed by :

· a first spur (17) which projects from the external face of the corresponding flap valve outwardly and upwardly from the middle point of its leading edge (8);

· a means about which said first spur (17) is articulated and which is fixed to the internal wall (4) of the base (2, 2a) ; and

· second and third spurs (18, 19) which project substantially from the lower edge of the base (2, 2a) which are oriented towards the axis of the base, namely the flow axis and slightly upwards, said second and third spurs (18, 19) being each disposed on each side of the articulation means of the first spur (17) and coming into contact with the leading edge (8) and the lower zone of the internal face of the corresponding flap valve, respectively in the open position and closed position.

3. Prosthetic heart valve according to claim 2, characterized in that said means for articulating said first spur is formed by a substantially horizontal and preferably substantially triangular stirrup (16) which projects from the internal wall (4) of the base (2, 2a) and which is disposed in the lower half of this wall, close to the median radial plane of the base, which stirrup has a radial width less than 25% of the radius of the base measured along the axis of symmetry of a flap valve.

4. Prosthetic heart valve according to claim 2, characterized in that said means for articulating said first spur is formed by two substantially horizontal spurs which project from the internal wall of the base and which converge towards each other, the free ends of these convergent spurs being slightly spaced apart and spaced away from the internal wall of the base by a radial distance less than 25% of the radius of the base measured along the axis of symmetry of a flap valve.

5. Prosthetic heart valve according to any one of claims 3 and 4, characterized in that the radial dimension of said means for articulating said first spur (17) is preferably between 10% and 15% of said radius of the base measured along the axis of symmetry of a flap valve.

6. Prosthetic heart valve according to any one of claims 1 to 5, characterized in that, in the open position, each flap valve (5, 6, 7) is substantially parallel to the blood flow axis, namely the internal and external faces of each flap valve are skimmed by the blood substantially parallel to the axis of the base.

7. Prosthetic heart valve according to any one of claims 1 to 5, characterized in that the internal wall (4) of the base has in axial cross section a curvilinear profile which comprises a concave upper portion (21), along which the thickness of said internal wall increases from the upper edge of the base, and a lower convex portion (22) along which the thickness of the internal wall decreases towards the lower edge of the base, said concave (21) and convex (22) portions of the axial profile of the internal wall of this base being separated by an annular line (25).

8. Prosthetic heart valve according to claim 7, characterized in that the separation line (25) between said concave (21) and convex (22) portions of said axial profile of the internal wall (4) of the base varies, in the zone between two contiguous flap valves (5-6; 6-7 ; 7-5), along a radial profile which projects with respect to the radial profile of the median zone of a flap valve and which is substantially symmetrical with respect to the trailing edges (9) of two contiguous flap valves touching each other in the closed position, the leading edge (8) of each flap valve having in its turn a profile adapted to said separation line (25) between said concave and convex portions (21 and 22) of the profile of the internal wall (4) of the base, namely the leading edge (8) of each flap valve has a central convex portion (8a) and two lateral re-entrant portions (8b), the latter being adapted in the closed position to the radial projecting profile of the internal wall of the valve, fitting in the closed position between the leading edge of each flap valve and the internal wall of the base taking place so as to reduce blood leaks to a minimum.

9. Prosthetic heart valve according to claim 8, characterized in that the portion (27) of said separation line (25) between said concave and convex portions (21, 22) of the axial profile of the internal wall (4) of the base, which corresponds to the convex portion (8a) of the leading edge (8) of each flap,valve (5, 6, 7) is situated at a distance from the lower edge of the base which is equal to about 10% of the height of this base, whereas the middle point of the portion (26) of said separation line (25) with projecting radial profile which corresponds to the two concave portions (8b) of two contiguous flap valves (5,6-6,7-7,5) is situated at a distance from the lower edge of the base which is equal to about 20% of its height, the passage between said two portions (26, 27) of said separation line (25) being progressive.

10. Prosthetic heart valve according to any one of claims 1 to 9, characterized in that each flap valve (5, 6, 7) has, at the level of its corners (11, 12) which come into contact with the internal wall (4) of the base in the closed position and on its external face, a reinforcement thickening (20) allowing the contact to be made by a surface and not by a point or a line, thus limiting the contact pressure, and so the wear, as well as the operating noise.

11. Prosthetic heart valve according to any one of claims 1 to 10, characterized in that each of the flap valves (5, 6, 7) is provided with closure advance means, namely means for anticipating closure of the flap valves in the return of the blood flow in the opposite direction, which cooperate with the closure beginning means formed by said virtual off-centred axes.

12. Prosthetic heart valve according to claim 11, characterized-in that said closure advance means are formed by magnetic masses of opposite polarities formed at the level of the corners of each flap valve, the magnetic polarities of the corners of two contiguous flap valves being opposite.

13. Prosthetic heart valve according to claim 12, characterized in that said magnetic masses formed at the level of the corners of each flap valve cooperate with additional magnetic masses which are disposed in the base between two contiguous flap valves and whose size is substantially equal to the distance which exists between the adjacent corners of two contiguous flap valves in the open position, each additional magnetic mass being disposed in the base so that its polarities correspond to the opposite polarities of the magnetic masses formed in the corners of the flap valves.

14. Prosthetic heart valve according to any one of claims 12 and 13, characterized in that said magnetic masses are formed by small magnets.

15. Prosthetic heart valve according to any one of claims 12 and 13, characterized in that said magnetic masses are formed by a magnetic material incorporated during manufacture of each flap valve or of the base.

16. Prosthetic heart valve according to any one of claims 1 to 15, characterized in that the base (2) is circular or substantially circular.

17. Prosthetic heart valve according to any one of claims 1 to 15, characterized in that the base (2a) is elliptic or substantially elliptic.

18. Prosthetic heart valve according to any one of claims 1 to 17, characterized in that the number of flap valves (5a, 6a) is equal to two.

19. Prosthetic heart valve according to any one of claims 1 to 17, characterized in that the number of flap valves (5, 6, 7) is greater than two.

20. Prosthetic heart valve according to claim 19, characterized in that the number of flap valves (5, 6, 7) is equal to three.

FIG.1

FIG.1 A

FIG.2

FIG.2 A

FIG.3

FIG.3 A

FIG.4

FIG.5

FIG.6E    FIG.6D    FIG.6C    FIG.6B    FIG.6A

FIG.6

FIG.7D    FIG.7C    FIG.7B    FIG.7A

17

16

17

A    B    C    D

7

A    B    C    D

FIG.7

FIG.8